# EUROPEAN PATENT APPLICATION

(11) **EP 2 055 265 A1**
(43) Date of publication of application: **06.05.2009**
(21) Application number: 08018326.2
(22) Date of filing: 20.10.2008
(51) Int. Cl.: A61F 2/16

(54) **Intraocular lens injection instrument**

(30) Priority: 01.11.2007 JP 2007285597
(71) Applicant: Nidek Co., Ltd., Gamagori-shi Aichi 443-0038 (JP)
(72) Inventor: Nagasaka, Shinji, Gamagori-shi Aichi,443-0038 (JP)
(74) Representative: Prüfer & Partner GbR European Patent Attorneys

(57) **Abstract**

An intraocular lens injection instrument (1) comprises: a lens holding part (10) for holding an intraocular lens (IOL) (40) having a pair of loop-shaped support parts (42) and for injecting the IOL in an eye through an incision formed in the eye; a main unit (20) having a cylindrical shape provided at a front end with the lens holding part; a push-out unit (30) axially movable in the cylindrical main unit to push the IOL out of the lens holding part, the push-out unit comprising: a push rod (31) for pushing out the IOL; and a head portion (50) provided at a distal end of the push rod, the head portion being contactable with the IOL; and a centering portion (60) fixedly formed in the main unit (20) and provided with an inner cylinder (61) configured to engage with the push rod, the inner cylinder having a front end which will be located right behind and in noncontact with a rear one of the support parts of the IOL when the IOL is held in the lens holding part, and the inner cylinder having a predetermined axial length needed to allow the push rod to engage in at least part of the inner cylinder while the head portion is in contact with the IOL.

## Description

### BACKGROUND OF THE INVENTION

### 1. Field of the Invention

The present invention relates to an injection instrument for injecting an intraocular lens into an eye.

### 2. Description of Related Art

As one of operative treatments for cataract, heretofore, there has been used a method in which a lens is removed from a patient's eye and then a foldable, soft intraocular lens (IOL) is injected in place of the lens. For injection of such foldable IOL, an IOL injection instrument called an injector is used to inject an IOL in a folded state into the eye through an incision. Thus, the incision has only to be formed in advance with a minimum diameter in the eye. This injector is configured to advance the IOL set therein by a push rod called a plunger while the IOL is folded into a smaller size, and push out the IOL. When the IOL is to be injected into the eye by the injector, the posture of the push rod of the plunger could not be controlled and hence the push rod is likely to wobble. Accordingly, the push rod may not push out the IOL appropriately. For avoiding such problem, an IOL injection instrument including a member for preventing deflection or displacement of a plunger has been proposed (see JP2004-24854A).

### SUMMARY OF THE INVENTION

However, the IOL injection instrument disclosed in JP'854A is provided with a posture control member placed at a front end of the plunger in interlocking relation thereto and arranged to prevent deflection. The interlocking relation will be released during lens movement. Such configuration causes an increase in the number of components and a complicated structure.

The present invention has an object to overcome the above problems and to provide an intraocular lens injection instrument capable of centering a push rod (a plunger) by a simple structure, thereby restraining deflection or displacement to appropriately push out an IOL.
Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

The object is attained by an intraocular lent injection instrument according to claim 1; specifically, an intraocular lens injection instrument comprises: a lens holding part for holding an intraocular lens (IOL) having a pair of loop-shaped support parts and for injecting the IOL in an eye through an incision formed in the eye; a main unit having a cylindrical shape provided at a front end with the lens holding part; a push-out unit axially movable in the cylindrical main unit to push the IOL out of the lens holding part, the push-out unit comprising: a push rod for pushing out the IOL; and a head portion provided at a distal end of the push rod, the head portion being contactable with the IOL; and a centering portion fixedly formed in the main unit and provided with an inner cylinder configured to engage with the push rod, the inner cylinder having a front end which will be located right behind and in noncontact with a rear one of the support parts of the IOL when the IOL is held in the lens holding part, and the inner cylinder having a predetermined axial length needed to allow the push rod to engage in at least part of the inner cylinder while the head portion is in contact with the IOL.
Further developments of the present invention are given in the dependent claims.

According to the present invention, the centering of a push rod (a plunger) can be made by a simple structure, thereby restraining deflection or displacement and appropriately pushing out an IOL.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.
In the drawings,
Figs. 1A and 1B are schematic external views of an IOL injection instrument in a preferred embodiment;
Fig. 2 is a schematic configuration view of an IOL;
Figs. 3A and 3B are schematic external views of the cartridge in the embodiment;
Figs. 4A and 4B are views showing a folding method of the IOL by transform of the cartridge;
Fig. 5 is a schematic perspective view of a cylindrical unit;
Fig. 6 is a side sectional view of the IOL injection instrument;
Fig. 7 is a schematic sectional view of a centering portion and its surrounding in which a head portion contacts with an optic part; and
Fig. 8 is a schematic sectional view of the centering portion and its surrounding in which the IOL is being pushed forward by the head portion.

### DETAILED DESCRIPTION OF THE PREFERRED EMBODIMENTS

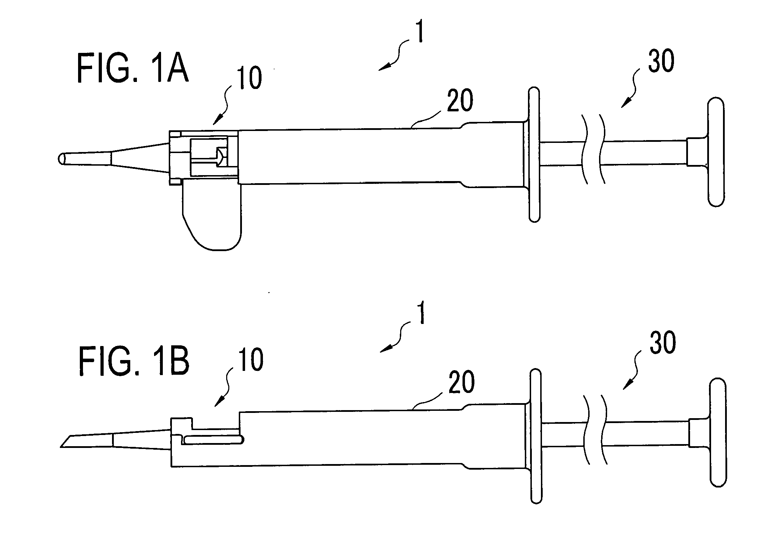

A detailed description of a preferred embodiment of the present invention will now be given referring to the accompanying drawings. Figs. 1A and 1B are schematic external views of an intraocular lens (IOL) injection instrument 1 used in the present embodiment; specifically, Fig. 1A is a plan view of the IOL injection instrument 1 and Fig. 1B is a side view of the same.

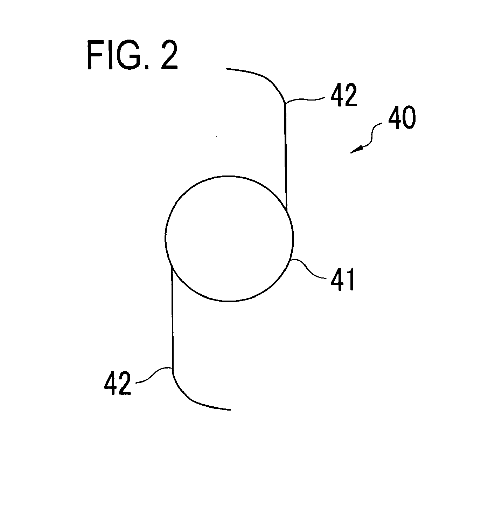

The IOL injection instrument 1 includes, in order of insertion into an eye, a lens holding part (hereinafter, referred to as a "cartridge") 10 including an injection part 11 which is inserted in an incision made in the eye and a setting part 12 in which an intraocular lens (IOL) 40 (see Fig. 2) is to be set, a cylindrical unit (an injection instrument main body) 20 in which the cartridge 10 is mounted at a front end, and a push-out unit (a push-out member or a plunger) 30 for pushing out the IOL 40 through a tip end of the cartridge 10 mounted in the cylindrical unit 20.

Fig. 2 is a schematic configuration view of the IOL 40. This IOL 40 includes an optic part 41 having predetermined refractive power and a pair of support parts 42 called loops for supporting the optic part 41 in the eye. The optic part 41 of the IOL 40 used in this embodiment is made of a material commonly used for a typical foldable soft intraocular lens, for example, a monomeric material such as hydroxyethyl methacrylate (HEMA) and a composite material of acrylic ester and methacrylate ester. The support parts 42 are also made of a material commonly used for a typical IOL support part such as polymethylmetacrylate (PMMA). The IOL 40 used in this embodiment is a three-piece IOL produced in such a way that the optic part 41 and the thin-loop-shaped support parts 42 are separately made of the aforementioned materials and then assembled together. An IOL to be used is not limited to the three-piece type and may be a one-piece type formed of an optic part and support parts integrally made in advance.

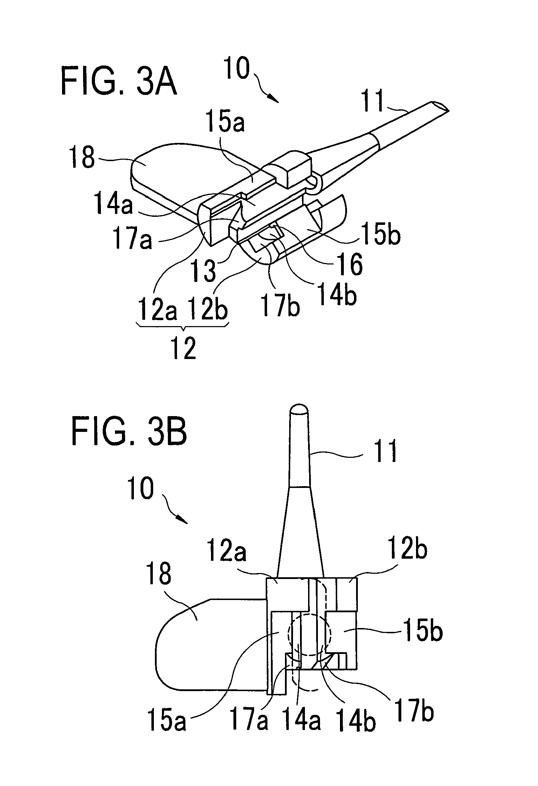

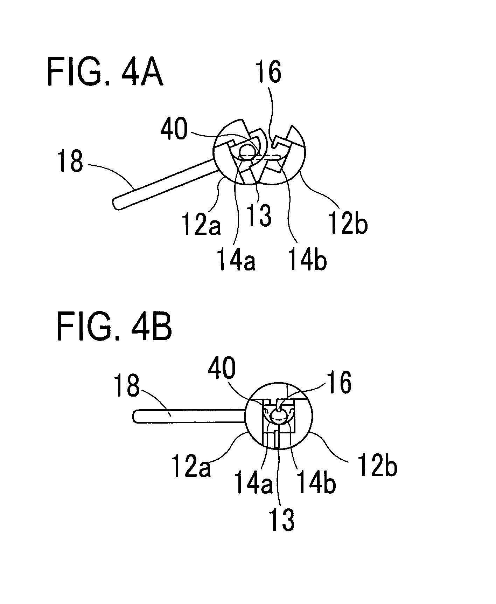

Figs. 3A, 3B, 4A and 4B are views showing the configuration of the cartridge 10. As shown in the figures, the cartridge 10 includes the injection part 11 having a tapered shape whose diameter is gradually smaller (thinner) toward the tip end and the setting part 12 for setting therein the IOL 40. The cartridge 10 is entirely made of synthetic resin as a disposable type which is to be thrown away after one use. Accordingly, the cartridge 10 is preferably made of resin or the like by molding.

The injection part 11 is formed in a hollow cylindrical shape through which the folded IOL 40 can be pushed out. The setting part 12 is constituted of two half-split elements 12a and 12b. As shown in Fig. 3A, the half-split elements 12a and 12b are connected to each other at respective lower edges by a hinge 13 so as to be opened and closed. The half-split elements 12a and 12b include setting stages 14a and 14b respectively for mounting thereon the IOL 40. The shapes (wall shapes) of the setting stages 14a and 14b are curved in a direction to fold the IOL 40.

When the half-split elements 12a and 12b are closed into close contact with each other, the shapes of walls (setting surfaces) of the setting stages 14a and 14b are transformed to substantially conform in cross section to the shape (semi-circle) of an opening of the injection part 11 on a rear end side (see Figs. 4A and 4B). The outer shape of the setting part 12 with the half-split elements 12a and 12b being closed is substantially equal to the shape of an inner wall of the cylindrical unit 20 mentioned later. As shown in Fig. 3B, the setting stage 14a (14b) is sized to allow the support part 42 which is a rear one (a lower one in Fig. 3B) (i.e., the one opposite from the injection part 11) to slightly extend out of the setting stage 14a when the optic part 41 (indicated by a broken line) of the IOL 40 is set on the setting stage 14a (14b).

In the IOL injection instrument 1 in this embodiment, the rear support part 42 of the IOL 40 is positioned on the left side with respect to an advancing direction of the IOL 40 when the IOL 40 is loaded in the cartridge 10. Furthermore, the total length of the half-split element 12a is determined to be longer than the total length of the half-split element 12b so that the half-split element 12a is longer on a rear end side of the cartridge 10. Such a difference in total length between the half-split elements 12a and 12b can provide space for receiving the support part 42 positioned on the rear side when the IOL 40 is loaded in the cartridge 10 so that the rear support part 42 is off the central axis of a push rod (a push axis) 31 mentioned later.

Covers 15a and 15b are provided as upper portions of the half-split elements 12a and 12b respectively to cover over the setting stages 14a and 14b when the half-split elements 12a and 12b are closed. The cover 15b is provided at an end with a protrusion 16 extending toward the setting stages 14a and 14b (the hinge 13). The protrusion 16 serves to restrict a folding direction of the IOL 40 set in the cartridge 10 to a direction along the inner walls (the setting surfaces) of the setting stages 14a and 14b.

Slant portions 17a and 17b are provided for facilitating insertion of the IOL 40 from the rear end side of the cartridge 10 onto the setting stages 14a and 14b. A grip 18 formed of a flat plate is provided on the side of the half-split element 12a. The grip 18 will be grasped by a user to hold the cartridge 10.

In the cartridge 10 having the above configuration, as shown in Fig. 4A, the IOL 40 is set on the setting stages 14a and 14b while the setting part 12 is opened (the half-split elements 12a and 12b are separated). Successively, the cartridge 10 is mounted in the cylindrical unit 20 while closing the half-split elements 12a and 12b as shown in Fig. 4B, thereby applying stress on the set IOL 40 to bend (fold) it.

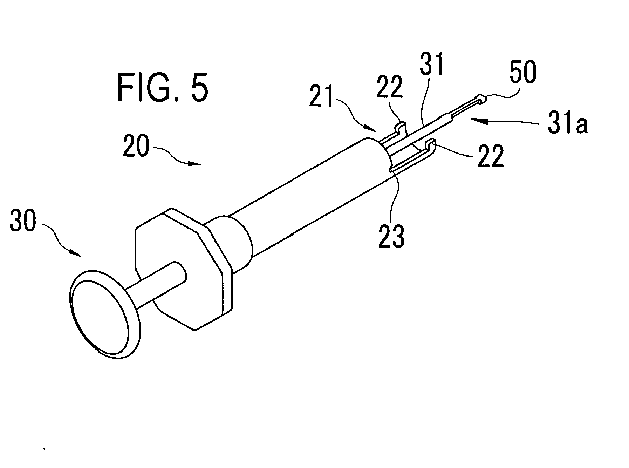

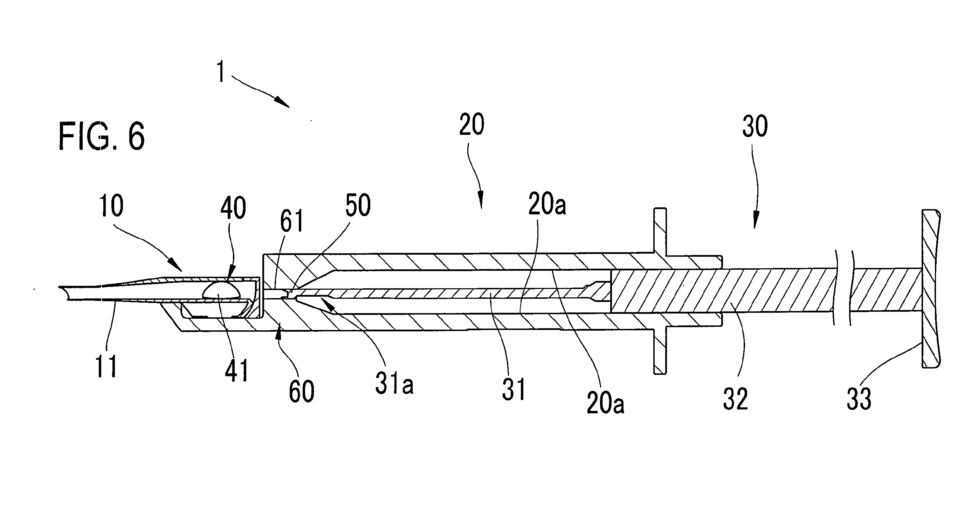

Fig. 5 is a schematic perspective view of the cylindrical unit 20. The internal structure of the cylindrical unit 20 is described referring to Fig. 6. As shown in the figures, the cylindrical unit 20 is provided in the distal end with a mounting part 21 in which the cartridge 10 is to be removably mounted. The mounting part 21 is of a nearly half split shape of the front end portion of the cylindrical unit 20. The mounting part 21 is formed with protrusions 22 at a front end and recesses 23 at a rear end, each of which are symmetric with respect to the push rod 31 extending along the central axis of the cylindrical unit 20. The protrusions 22 are positioned slightly above the central axis of the cylindrical unit 20 and have a distance therebetween slightly narrower (shorter) than the inner diameter of the cylindrical unit 20. Each protrusion 22 has a shape for a snap-in configuration serving as a guide for causing the opened half-split elements 12a and 12b to move in a closing direction when the cartridge 10 is mounted in the mounting part 21 and limiting the width of the half-split elements 12a and 12b, and also for reliably holding the mounted cartridge 10 against coming-off the cylindrical 20. Right and left edges of the mounting part 21 also serve as a guide as with the protrusions 22 to cause the half-split elements 12a and 12b to close. The push rod 31 is formed at its tip end with a head portion 50 (the details thereof will be mentioned later) which will contact with (catch) the optic part 41 of the IOL 40. A narrow portion 31a (the details thereof will be mentioned later) of a shaft shape having a smaller diameter than the push rod 31 is formed between the head portion 50 and the push rod 31.

Next, the internal structure of the IOL injection instrument 1 assembled from the cartridge 10 and the cylindrical unit 20 is explained below. Fig. 6 is a schematic sectional view of the IOL injection instrument 1. In this figure, the cartridge 10 is mounted in the mounting part 21 and thus the IOL 40 is placed in a folded state.

As shown in Fig. 6, the inside of the cylindrical unit 20 is hollow, in which the push-out unit 30 is inserted so as to be movable forward and backward in an axial direction of the cylindrical unit 20. This push-out unit 30 includes the push rod 31, a base part 32, and a press part 33. The cylindrical unit 20 and the push-out unit 30 are a disposable type as with the cartridge 10, which will be thrown away after one use. Accordingly, each of the units 20 and 30 is integrally made of resin or the like by molding. In Fig. 6, the support parts 42 are not illustrated for the sake of convenience.

The press part 33 which will be pressed by an operator or surgeon to push out the IOL 40 and the base part 32 integrally connected with the end of the press part 33 have rigidity enough to keep their shapes even when they are pressed by the operator or others and to perform appropriate centering by pressing as mentioned later. The base part 32 is therefore designed to have as large a diameter as possible. In this embodiment, the base part 32 is made with a diameter equal to the inner diameter of an inner wall 20a of the cylindrical unit 20. The base part 32 is less likely to wobble even when it moves forward and backward within the cylindrical unit 20. The base part 32 and the inner wall 20a have mirror-finished surfaces to reduce friction or the like caused by the forward and backward movement of the push-out unit 30. As above, a centering mechanism for the push rod 31 is configured. A base (a boundary between the push rod 31 and the base part 32) of the push rod 31 is centered (positioned).

The push rod 31 is formed of an axial rod having a smaller diameter than the base part 32 and is connected to a front end of the base part 32. When the base part 32 is moved forward, the IOL 40 is pushed forward from the cartridge 10 mounted in the distal end of the cylindrical unit 20. The push rod 31 (the push-out unit 30) serves to deliver the IOL 40 out of the tip end of the cartridge 10. The push rod 31 is designed to have a diameter capable of passing through the injection part 11 and the cartridge 10 and smaller than the base part 32. A distal end portion of the push rod 31 includes the narrow portion 31a having a smaller diameter and a predetermined axial length to prevent the rear loop (the support part 42) of the IOL 40 set in the cartridge 10 from tangling (becoming caught) between the push rod 31 and a passage and hence becoming damaged when the IOL 40 is to be pushed out. In this embodiment, the length of the narrow portion 31a is almost equal to the length of the support part 42 when spread in the cartridge 10 in association with movement of the optic part 41 in a process of moving the push rod 31. Furthermore, the head portion 50 for catching the IOL 40 is formed at a front end (an extreme forward end) of the narrow portion 31a. The narrow portion 31a has a length needed to avoid interference with the rear support part 42 (i.e., tangling of the support part 42) during push-out of the IOL 40.

The head portion 50 is designed to have a wedge-shaped cross section capable of easily holding or catching the optic part 41 so as to pick up the optic part 41. The diameter of the head portion 50 is determined to be equal to or smaller than the push rod 31. With the above configuration that the push rod 31 is smaller in diameter than the base part 32 and the narrow portion 31a is smaller in diameter than the push rod 31, the details of which will be mentioned later, the space is produced for receiving the support part 42 between the push rod 31 and the passage (the inside of the cartridge 10 and others) during a push-out operation of the IOL 40 mentioned later, thereby preventing damages from the push-out unit 30.

A centering portion 60 is fixedly formed in the cylindrical unit 20 in a position as close as possible to the distal end of the cylindrical unit 20 (around the recesses 23 in this embodiment). The centering portion 60 has a predetermined thickness (corresponding to a distance Y mentioned later) in an axial direction. As shown in the figure, a back end portion of the centering portion 60 is formed in a hollow cone shape whose inner diameter is gradually smaller toward a front end portion in which an inner cylinder 61 is formed with a diameter allowing the push rod 31 to engage in the inner cylinder 61. Accordingly, the front end of the inner cylinder 61 will be located right behind the rear support part 42 of the IOL 40 when held in the cartridge 10 (setting stages 14a and 14b). The centering portion 60 having such a hollow cone shape facilitates engagement of the push rod 31 in the inner cylinder 61 during the push-out operation. When the push rod 31 engages in the inner cylinder 61, the push rod 31 is aligned in the cylindrical unit 20 and hence centered therein. Thus, deflection of the push-rod 31 can be prevented. The centering portion 60 serves to restrict forward movement of the base portion 32 during the push-out operation of the IOL 40. This makes it possible to prevent the head portion 50 from excessively moving out of the injection part 11 during the push-out operation. Accordingly, the IOL 40 can be injected in the eye appropriately. The centering portion 60 is integrally molded with the cylindrical unit 20.

The inner cylinder 61 has a nearly semi-circular shape in cross section. The push rod 31 which will engage in the inner cylinder 61 also has a semi-circular shape in cross section equal to the inner cylinder 61. The push rod 31 is therefore held against rotation (torsional rotation about the central axis of the push rod) during the push-out operation.

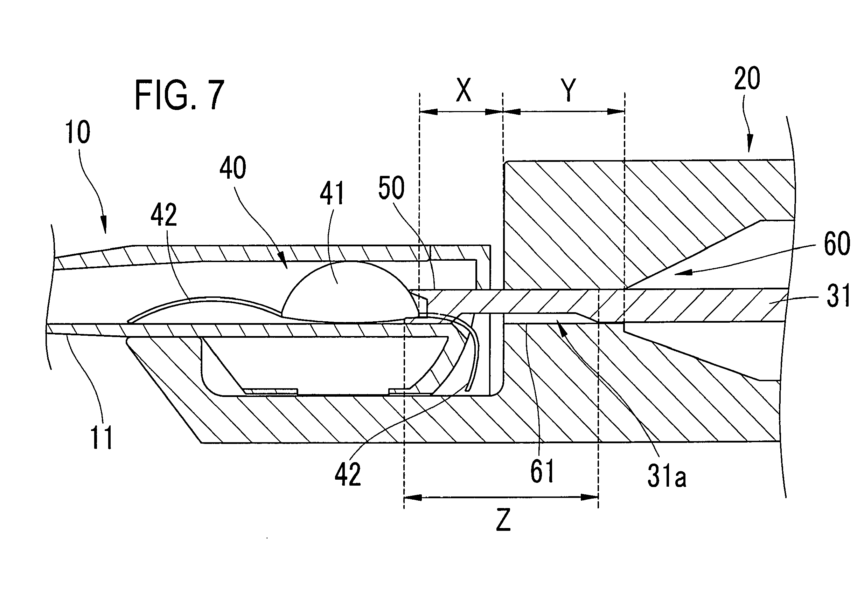

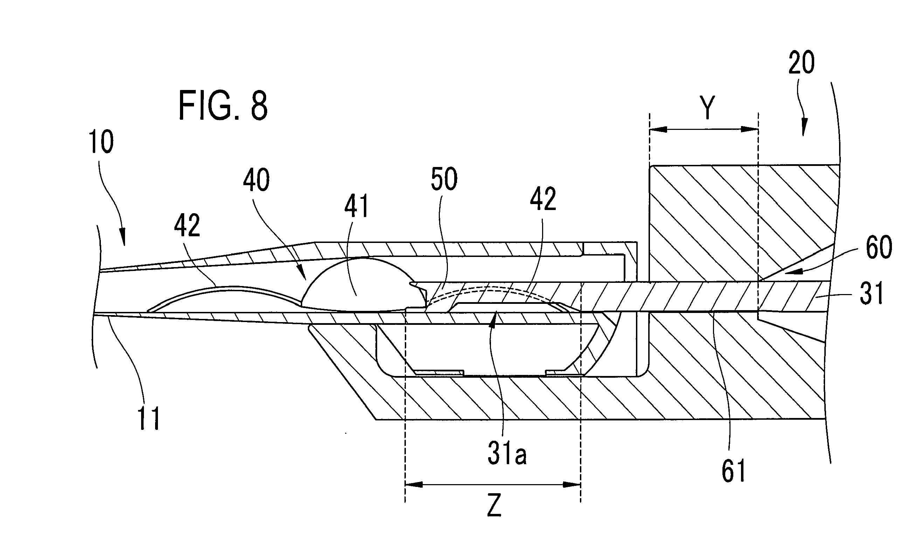

The following explanation is given to the centering of the push rod 31 in association with the push-out operation of the IOL 40 (a process of moving the push rod 31). Fig. 7 is a schematic sectional view of the centering portion 60 and its surrounding in a state where the push rod 31 starts to engage in the centering portion 60. Fig. 8 is a schematic sectional view of the centering portion 60 and its surrounding in another state where the head portion 50 contacts with the optic part 41. The support part 42 in Figs. 7 and 8 is illustrated on the sectional view for the shake of simplicity and a portion of the support part 42 overlapping the head portion 50 and the narrow portion 31a is shown by a broken line. When the IOL 40 is folded inside the cartridge 10, the front support part 42 is spread forward by the inner wall of the cartridge 10. On the other hand, the rear support part 42 hangs down, without stress, from the rear end (the base end) of the cartridge 10. In this state, preferably, the rear support part 42 is out of contact with the centering portion 60.

In the figure, assuming that the distance from the rear end of the IOL 40 (the optic part 41) set in the cartridge 10 to the front end of the centering portion 60 is X. This distance X is a distance determined to ensure a predetermined amount of release (space) of the rear support part 42 when the IOL 40 is folded. In the case of a short distance X, when the cartridge 10 is mounted, the rear support part 42 is likely to excessively contact with the rear wall (the front end face of the centering portion 60) and be damaged. Accordingly, the distance X is determined as such a distance as not to largely change a naturally curved form of the support part 42.

Assuming the length (distance) of the inner cylinder 61 is Y. This distance Y is to control the timing the push rod 31 engages in the inner cylinder 61 during the push-out operation. The distance Y is so long as to allow the narrow portion 31a to pass through the inner cylinder 61 and also the push rod 31 engageable in the inner cylinder 61 to enter the inner cylinder 61 from an entrance (on a back end side) just before or when the head portion 50 reaches the optic part 41 during the push-out operation. At that time, the push rod 31 is not required to engage in the entire passage of the inner cylinder 61. The push rod 31 has only to partly engage in at least a region around the entrance of the inner cylinder 61. The shape of the inner cylinder 61 is determined to allow engagement of the push rod 31 during the push-out operation of the IOL 40.

Assuming the distance (length) from the tip end of the head portion 50 to a rear end of the narrow portion 31a is Z. This distance Z is determined to produce the space for setting the support part 42 free when spread. As shown in Fig. 8, the distance Z is determined to be long enough to provide the space formed around the narrow portion 31a (herein, the space under the narrow portion 31a) for receiving the support part 42 spread in association with the push-out operation in order to prevent the spread support part 42 from contacting (interfering) with the push rod 31 in moving. If the distance Z is too long, the narrow portion 31a is likely to be warped, causing deflection of the head portion 50 even when the centering of the push rod 31 is accurately made. Therefore, the distance Z is preferably determined as a length as short as possible but enough to set the support part 42 free.

The centering portion 60 is therefore preferably located in the cylindrical unit 20 and apart from the aforementioned centering mechanism. With such configuration, the centering accuracy of the push-out unit 30 (the head portion 50) can be enhanced. Thus, the relationship of X, Y, and Z is determined by an expression of Z-X≤Y.

With the above configuration, a centering sequence is carried out during the push-out operation of the IOL 40. The push rod 31 is first centered by the centering mechanism as mentioned above. In this state, when the push rod 31 is moved forward, the push rod 31 engages in the inner cylinder 61. Accordingly, the push rod 31 is centered at two points by the centering mechanism and the centering portion 60. At that time, the head portion 50 comes into contact with the optic part 41. When the push rod 31 is further moved forward, the push rod 31 remains centered and the optic part 41 is caught by the head portion 50 and pushed into the injection part 11.

As mentioned above, the IOL can appropriately be pushed out by a simple structure without increasing the number of components, and the deflection can be restrained. Furthermore, the IOL can appropriately be pushed out without using a member which is moved in interlocking relation to the push rod for centering and then is separated therefrom.

In the above embodiment, the inner cylinder 61 is formed to have a fixed sectional shape engageable with the push rod 31 by the distance Y. The shape of the inner cylinder 61 is however not limited thereto. Any configuration may be adopted if only the centering of the push rod 31 is performed in a position at the distance Y apart from the front end of the centering portion 60. It is only necessary to provide a member in a position at the distance X apart from the rear end of the optic part 41 so that the member will contact with the support part 42 to such an extent that does not apply stress thereon or to provide a member in a position near the support part 42 without contacting therewith. Accordingly, a thin engagement member (an inner cylinder) may be provided in each of the front end portion and the back end portion of the centering portion 60.

Operations of the IOL injection instrument 1 having the above configuration will be explained below. A user (a surgeon) grasps the grip 18 of the cartridge 10 by one hand to hold the cartridge 10 and picks up the IOL 40 with forceps by the other hand. The user inserts the picked IOL 40 into the cartridge 10 from the rear end thereof so as to put it on the setting stages 14a and 14b. While no stress is applied on the cartridge 10, the half-split elements 12a and 12b are in an open state as shown in Fig. 4A. Accordingly, the IOL 40 set on the setting stages 14a and 14b is retained in an unfolded state (a state where no stress is applied thereon) in the cartridge 10.

To mount the cartridge 10 in the cylindrical unit 20, firstly, the push rod 31 is pulled toward the rear end of the cylindrical unit 20. The user then engages the grip 18 (the rear end) of the cartridge 10 in the recess 23 of the mounting part 21 and pushes down the setting part 12 (the half-split elements 12a and 12b) into the mounting part 21 so as to press the bottom of the mounting part 12 against the protrusions 22 (or the right and left edges of the mounting part 21). When the bottom (the lower part) of the setting part 12 is pressed against the protrusions 22 (or the right and left edges of the mounting part 21), the protrusions 22 will guide the half-split elements 12a and 12b to close into contact with each other. As the setting part 12 is further pushed into the mounting part 21, the half-split elements 12a and 12b are mounted in a closed state in the mounting part 21 as shown in Fig. 4B.

In such a state where the half-split elements 12a and 12b are closed in contact with each other, the width (interval) of the setting stages 14a and 14b is narrow so that the wall surfaces of the setting stages 14a and 14b push the IOL 40 from right and left. The IOL 40 receives stress and thus is folded along the wall surfaces of the setting stages 14a and 14b.

After the cartridge 10 is mounted in the mounting part 21, the user inserts the injection part 11 in the patient's eye from which a lens has been removed in advance and then pushes the press part 33 to move the base part 32 and the push rod 31 forward. The head portion 50 and the narrow portion 31a pass through the centering portion 60 and the push rod 31 engages in the inner cylinder 61 of the centering portion 60. Thus, the push rod 31 is centered and the head portion 50 integrally formed with the push rod 31 is moved forward to push the IOL 40 by holding the edge of the optic part 41 toward the injection part 11. When the IOL 40 enters the injection part 11 and advances in the injection part 11 whose inner diameter becomes narrower, the IOL 40 is gradually folded (rounded) along the inner wall surface of the injection part 11. At this time, the support parts 42 are spread out. When the press part 33 is further pressed forward, the IOL 40 is pushed out of the tip end of the injection part 11.

It is to be noted that the above embodiment exemplifies the configuration that the centering portion is formed with the inner cylinder having the same cross section as the push rod to enhance the centering accuracy during the push-out operation of the IOL, thereby enabling appropriate push-out of the IOL. However, the invention is not limited to such configuration. The push rod and the centering portion may be configured in any form if only it does not cause axial rotation of the push rod and can perform appropriate centering. For instance, the push rod and the centering portion may be provided with a protrusion and a groove which slidably engage with each other.

In the above embodiment, the IOL 40 folded in the cartridge 10 is centered by the centering portion 60 and others and is pushed out from the injection part 11 by the push-out unit 30; however, the invention is not limited thereto. For instance, it may be arranged to push the IOL with no stress applied, by the push-out unit 30 having been centered, thereby folding the IOL 40 by the inner configuration of the cartridge 10 to push the IOL out of the injection part 11.

The above embodiment is configured such that the narrow portion is provided between the head portion and the push rod, but it is not limited thereto. As an alternative, the push-out unit may be provided with no narrow portion. In this case the head portion is placed in contact with the support part to push out the intraocular lens. For instance, in the case of pushing out an intraocular lens of one-piece type with high-strength support parts, the support parts are not required to be held under the push rod. In this case, the inner cylinder of the centering portion has only to have a predetermined axial length needed to allow the push rod to engage in at least part of the inner cylinder while the head portion is in contact with the intraocular lens.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens injection instrument (1) comprising:
a lens holding part (10) for holding an intraocular lens (IOL) (40) having a pair of loop-shaped support parts (42) and for injecting the IOL in an eye through an incision formed in the eye;
a main unit (20) having a cylindrical shape provided at a front end with the lens holding part;
a push-out unit (30) axially movable in the cylindrical main unit to push the IOL out of the lens holding part, the push-out unit comprising:
a push rod (31) for pushing out the IOL; and
a head portion (50) provided at a distal end of the push rod, the head portion being contactable with the IOL; and
a centering portion (60) fixedly formed in the main unit (20) and provided with an inner cylinder (61) configured to engage with the push rod, the inner cylinder having a front end which will be located right behind and in noncontact with a rear one of the support parts of the IOL when the IOL is held in the lens holding part, and the inner cylinder having a predetermined axial length needed to allow the push rod to engage in at least part of the inner cylinder while the head portion is in contact with the IOL.

2. The intraocular lens injection instrument according to claim 1, wherein
the push-out unit comprises a narrow portion (31a) having a shaft shape formed between the push rod (31) and the head portion (50), the narrow portion having a predetermined axial length and a diameter smaller than a diameter of the push rod to prevent interference with the rear one of the support parts, and
the inner cylinder has an inner diameter allowing the narrow portion to pass therethrough and the push rod to engage therein when the head portion comes contact with the IOL in a process of moving the push-out unit.

3. The intraocular lens injection instrument according to claim 1 or 2, wherein the narrow portion has the axial length almost equal to a length of the support part which will be spread in the lens holding part in association with movement of an optic part (41) of the IOL in the process of moving the push rod.

4. The intraocular lens injection instrument according to one of claims 1 to 3, wherein the inner cylinder has a sectional shape in an axial direction engageable with the push rod by at least a predetermined axial length.

5. The intraocular lens injection instrument according to one of claims 1 to 4, further comprising a mounting part (21) in which the lens holding part is removably mounted.
